(19) Europäisches Patentamt
European Patent Office
Office européen des brevets

(11) Numéro de publication : **0 337 890 B1**

(12) # FASCICULE DE BREVET EUROPEEN

(45) Date de publication du fascicule du brevet : **18.03.92 Bulletin 92/12**

(51) Int. Cl.$^5$ : **C07J 9/00,** A61K 31/575, A61K 7/48

(21) Numéro de dépôt : **89401023.0**

(22) Date de dépôt : **13.04.89**

(54) **Complexes à base d'anthraline et d'un stérol, leur procédé d'obtention et leur utilisation en thérapeutique et cosmétique.**

(30) Priorité : **14.04.88 LU 87201**

(43) Date de publication de la demande : **18.10.89 Bulletin 89/42**

(45) Mention de la délivrance du brevet : **18.03.92 Bulletin 92/12**

(84) Etats contractants désignés : **AT BE CH DE ES FR GB GR IT LI NL SE**

(56) Documents cités : **GB-A- 2 107 589**

(73) Titulaire : **CENTRE INTERNATIONAL DE RECHERCHES DERMATOLOGIQUES GALDERMA - CIRD GALDERMA Groupement d'Intérêt Economique dit: Sophia Antipolis F-06560 Valbonne (FR)**

(72) Inventeur : **Shroot, Braham Villa 35 Hameaux de Val-Bosquet Chemin de Val-Bosquet F-06600- Antibes (FR)**
Inventeur : **Caron, Danièle 3, Rue de Boyère Haut Sartoux F-06560-Valbonne (FR)**
Inventeur : **Caron, Jean-Claude 3, Rue de Boyère Haut Sartoux F-06560-Valbonne (FR)**
Inventeur : **Brzokewicz, Alain Les Jonquilles Bâtiment 4 - Les Semboules F-06600 - Antibes (FR)**

(74) Mandataire : **Stalla-Bourdillon, Bernard et al CABINET NONY & CIE 29, rue Cambacérès F-75008 Paris (FR)**

EP 0 337 890 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des complexes à base d'anthraline et d'un stérol, leur procédé d'obtention et leur utilisation en thérapeutique et cosmétique. Les principales indications concernent le traitement des affections inflammatoires de la peau, des ongles et du cuir chevelu, notamment le traitement du psoriasis, de l'eczéma et des verrues.

Parmi les substances particulièrement actives dans le traitement du psoriasis, l'anthraline ou dithranol demeure, malgré ses inconvénients, le traitement de choix compte-tenu de ses résultats.

Les principaux inconvénients de l'anthraline sont essentiellement son manque de stabilité qui provoque la formation de taches brunâtres sur la peau et les vêtements et son pouvoir irritant de la peau.

L'anthraline est en effet particulièrement instable et se dégrade en donnant par auto-oxydation du dimère de l'anthraline (ou 1,8-1′,8′-tétrahydroxy-10,10′ dianthranone) et par oxydation d'autres produits de dégradation dont la 1,8-dihydroxy 9,10-anthraquinone.

Certains produits de dégradation sont inactifs dans la chimiothérapie du psoriasis et sont essentiellement responsables des irritations et des taches de la peau et des vêtements.

De nombreuses études ont été entreprises en vue d'améliorer la stabilité de l'anthraline et celles-ci on conduit à diverses propositions.

Parmi ces propositions, on peut notamment citer l'utilisation d'agents stabilisants tels que ceux décrits dans les brevets US n°4.287.214 et 4.367.224 ou l'utilisation de certains véhicules notamment des esters d'acides gras tels que ceux décrits dans le brevet US 4.316.902 et les demandes de brevets français n°82.01327 et 84.13431 et anglais GB.A. 2 107 589.

Ces diverses solutions se sont toutefois révélées à l'usage ne pas être totalement satisfaisantes, les agents stabilisants ne permettant pas une conservation prolongée dans le temps et les nouveaux véhicules ne favorisant pas une bonne pénétration de l'anthraline à travers la peau.

Il résulte de ce qui précède que si l'on peut agir de façon satisfaisante sur la stabilité de l'anthraline, on doit pouvoir résoudre simultanément les problèmes soulevés par l'irritation et la formation des taches sur la peau et sur les vêtements.

Après de nombreuses études, on vient de constater que la stabilisation recherchée de l'anthraline pouvait être obtenue en formant des complexes avec certains stérols.

Il a en effet été montré que l'anthraline, de par sa structure chimique, possède des liaisons hydrogène intramoléculaires entre les groupements hydroxyles 1,8 et le carbonyle en position 9.

Ces liaisons hydrogène agissent comme stabilisatrices de cette molécule. En effet, si elles sont supprimées, par exemple dans un solvant polaire, il se produit une activation de la position $C_{10}$ conduisant à une décomposition rapide de la molécule.

Le maintien de liaisons hydrogène serait donc un moyen pour améliorer la stabilité de l'anthraline. Ceci a été atteint selon la présente invention par la création d'interactions électrostatiques dans des complexes anthraline-stérols. L'examen des spectres obtenus par réflexion dans le proche infra-rouge semble confirmer l'existence de ces interactions.

Il s'est en effet avéré que les complexes selon l'invention présentaient une excellente stabilité dans le temps même lorsque le support n'était pas anhydre et que par ailleurs les propriétés pharmacologiques de l'anthraline étaient conservées et même renforcées.

En ce qui concerne l'activité des complexes selon l'invention, il est par ailleurs possible que le stérol potentialise les effets de l'anthraline et agisse sur les inflammations de la peau.

La présente invention a donc pour objet à titre de produit industriel nouveau un complexe à base d'anthraline et d'un stérol, le complexe ayant un point de fusion inférieur de 10 à 25°C à celui du constituant du complexe fondant à la température la plus basse et la proportion d'anthraline variant entre 10 et 90% molaire.

Le stérol est de préférence choisi parmi:

- le cholestérol,
- les dérivés mono et polyhydroxylés ou carboxylés sur la chaîne latérale en $C_{17}$ du cholestérol et leurs alkyl-esters inférieurs ($C_1$-$C_6$) et notamment le 25-hydroxy-cholestérol, le 20-hydroxy-cholestérol et l'ester méthylique de l'acide (3-hydroxychol-5-ène)-24-oïque,
- les dérivés obtenus par isomérisation et/ou création d'insaturations dans la chaîne latérale en $C_{17}$ du cholestérol et notamment le stigmastérol, et
- les esters jusqu'à 12 atomes de carbone et éthers jusqu'à 6 atomes de carbone en position $C_3$ du cholestérol et notamment l'acétate de cholestérol.

Comme indiqué ci-dessus les proportions d'anthraline dans le complexe peuvent varier, selon le dérivé du cholestérol, entre 10 et 90% molaire, ces proportions pour chacun des complexes étant toutefois susceptibles de faibles variations de l'ordre de ± 0,05%M.

Parmi les différents complexes particulièrement préférés selon l'invention, on peut mentionner les suivants:

(i) Anthraline (0,26%M) - cholestérol (0,74%M)

(ii) Anthraline (0,70%M) - 25-hydroxy-cholestérol (0,30%M)

(iii) Anthraline (0,30%M) - Stigmastérol (0,70%M)

(iv) Anthraline (0,42%M) - Acétate de cholestérol (0,58%M)

(v) Anthraline (0,17%M) - 20-hydroxy-cholestérol (0,83%M)

(vi) Anthraline (0,35%M) - Ester méthylique de l'acide (3-hydroxychol-5-ène)-24-oïque (0,65%M)

La détermination des proportions molaires pour chacun des complexes a été effectuée par analyse thermique différentielle à partir de la détermination d'un diagramme de phases binaires.

Par extrapolation, on détermine les proportions exactes de chacun des constituants, le complexe ainsi défini apparaissant comme un produit pur lors de la détermination de sa pureté par analyse thermique différentielle.

Les différents complexes selon l'invention se présentent sous forme d'une poudre plus ou moins fortement colorée en jaune et se caractérisent par leur point de fusion qui est inférieur de 10 à 25°C à celui du constituant fondant à la température la plus basse.

A titre d'exemple les points de fusion des complexes cités ci-dessus sont les suivants:

Anthraline-cholestérol : 132° ± 2°C,

Anthraline - 25-hydroxy-cholestérol : 157° ± 2°C,

Anthraline - stigmastérol : 150° ± 2°C,

Anthraline - acétate de cholestérol: 102° ± 2°C

Anthraline - 20 - hydroxy-cholestérol : 118° ± 2°C

Anthraline - Ester méthylique de l'acide (3-hydroxychol-5-ène)-24-oïque : 126° ± 2°C

Le point de fusion de l'anthraline et des stérols de ces complexes étant les suivants:

Anthraline : 176-180°C

Cholestérol : 148-149°C

25-hydroxy-cholestérol: 178-180°C

Stigmastérol: 170°C

Acétate de cholestérol: 115-116°C

20-hydroxy-cholestérol: 128-129°C

Ester méthylique de l'acide (3-hydroxychol-5-ène)-24-oïque : 143-144°C

L'analyse des complexes selon l'invention, par réflexion dans le proche infra-rouge, sur un appareil InfraALYZER de la Société TECHNICON, a permis de distinguer sans ambiguité les complexes selon l'invention des différents mélanges physiques correspondants.

Selon cette méthode d'analyse un échantillon, placé dans une cellule de mesure spécifique est soumis, par l'intermédiaire d'un système optique approprié, à un rayonnement infra-rouge de longueurs d'onde variant, selon l'appareil, entre 700 et 2600nm (modèle InfraALYZER 450, 19 longueurs d'onde sélectionnées par filtres) et entre 700 et 3800nm (modèle InfraALYZER 500, monochromateur utilisant toute la gamme spectrale). La lumière réfléchie par cet échantillon est concentrée par une sphère d'intégration et analysée par un photorécepteur approprié.

A chaque longueur d'onde émise est associée une valeur d'absorbance par l'échantillon. Le traitement statistique de ces résultats permet de faire des dosages qualitatifs et quantitatifs des complexes considérés à la fois à l'état pur et en formulation.

Pour chaque identification d'un complexe on utilise comme produits de référence l'anthraline, le stérol concerné et leur mélange physique aux proportions molaires du complexe.

La présente invention a également pour objet le procédé de préparation des complexes tels que définis ci-dessus.

Ce procédé consiste à chauffer, sous agitation et sous azote, un mélange d'anthraline et du stérol, dans les proportions molaires ci-dessus mentionnées déterminées par analyse thermique différentielle, à une température voisine de celle du point de fusion du composé qui a la température de fusion la plus élevée, généralement de 5 à 10°C supérieure, à maintenir cette température pendant un temps compris entre environ 10 et 20 minutes et à laissser refroidir lentement à la température ambiante. Le produit solide obtenu est alors broyé sous forme d'une poudre.

La présente invention a en outre pour objet une composition pharmaceutique ou cosmétique destinée au traitement de la peau, des ongles ou du cuir chevelu, cette composition contenant, en tant que substance active, au moins un complexe anthraline-stérol tel que défini ci-dessus en une proportion comprise entre 0,5 et 10% en poids, dans un véhicule pharmaceutiquement ou cosmétiquement acceptable.

Les compositions selon l'invention sont de préférence des onguents à base de vaseline, des émulsions du type eau-dans-l'huile ou peuvent également se présenter sous forme d'un film dans lequel le complexe insoluble est dispersé ou encore sous forme sèche.

Les émulsions peuvent contenir comme agent épaississant un silicone élastomère du type polyvinyldiméthylsiloxane associé à une charge de silice.

Les silicones élastomères peuvent être représentés par la formule suivante:

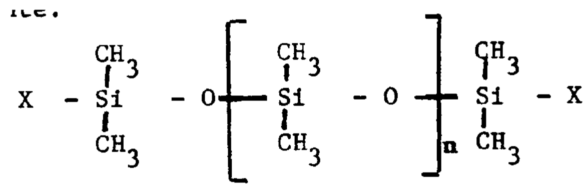

dans laquelle:

X représente OH et n est tel que la viscosité du silicone élastomère associé à la charge de silice soit comprise entre environ 650 et 1150cps mesurée à 24°C (contraves).

De tels produits sont notamment décrits dans le Brevet français BSM 8424M.

Comme silicone élastomère préféré, on peut en particulier mentionner le produit vendu par la Société DOW CORNING CORPORATION sous la dénomination de "DOW CORNING MDX-4-4210" qui se présente sous forme d'un kit constitué d'une part d'un produit de base (silicone élastomère + silice) et d'autre part d'un agent de réticulation ou catalyseur de vulcanisation, le produit de base, étant seul utilisé pour constituer l'agent épaississant ou bien encore additionné à son agent de réticulation lorsque l'on souhaite obtenir formulation une sous forme d'un film.

Ces compositions peuvent également contenir un anti-oxydant du type acide salicylique ou butylhydroxytoluène dans une proportion < à 0,5%.

Dans une étude clinique d'évaluation du complexe anthraline-cholestérol dans le traitement du psoriasis, des onguents à 1,7 et à 5,7% de ce complexe ont été comparés à des préparations hospitalières d'anthraline ayant des concentrations équivalentes.

Dans les conditions de l'étude, les formulations au complexe anthraline-cholestérol sont efficaces et parfaitement tolérées par les patients. Le temps moyen nécessaire pour obtenir un blanchiment de la peau est de 19,3 jours pour les complexes et de 27,1 jours pour les préparations hospitalières. La tolérance des formulations au complexe anthraline-cholestérol, meilleure que celle des préparations hospitalières, apparaît comme remarquable (absence de sensations de brûlures cutanées, pigmentation cutanée réduite et absence de taches sur la peau et les vêtements).

On va maintenant donner à titre d'illustration et sans aucun caractère limitatif des exemples de préparation des complexes selon l'invention ainsi que des exemples de compositions pharmaceutiques et cosmétiques.

## PREPARATION DES COMPLEXES

### Préparation du complexe anthraline (0,26M) - cholestérol (0,74M).

On introduit dans un ballon, muni d'un agitateur et d'une arrivée d'azote, 10g (0,044mole) d'anthraline et 48,83g (0,126mole) de cholestérol en poudre. On porte lentement l'ensemble à 190°C et on maintient à cette température pendant 15mn. On laisse refroidir lentement jusqu'à température ambiante tout en maintenant l'agitation et le courant d'azote. Après broyage le complexe attendu se présente sous forme d'une poudre jaune dont le point de fusion est de 132° ± 2°C.

Selon le même mode opératoire que celui décrit ci-dessus on a préparé les complexes suivants:

(a) Anthraline (0,42M) - Acétate de cholestérol (0,58M)
Point de fusion = 102° ± 2°C,

(b) Anthraline (0,30M) - Stigmastérol (0,70M)
Point de fusion = 150° ± 2°C,

(c) Anthraline (0,70M) - 25-hydroxy-cholestérol (0,30M)
Point de fusion = 157° ± 2°C.

(d) Anthraline (0,17%M) -20-hydroxy-cholestérol (0,83%M)
Point de fusion = 118° ± 2°C

(e) Anthraline (0,35%M) -Ester méthylique de l'acide (3-hydroxychol-5-ène)-24-oïque (0.65% M)
Point de fusion = 126° ± 2°C

EXEMPLE 1 - ONGUENT

- Complexe anthraline-cholestérol......... 5,68g
- Vaseline.................................94,32g

Cet onguent est préparé à froid par mélange de 5,68g du complexe anthraline-cholestérol et 94,32g de vaseline blanche. Après mélange et homogénéisation au broyeur tricylindrique, l'onguent est alors conditionné en tube ou en pot.

On n'a observé aucune dégradation de la composition après stockage pendant 12 mois à 37°C.

EXEMPLE 2 - EMULSION EAU-DANS-L'HUILE

- Silicone élastomère "Dow Corning MDX-4-4210"............... 38,51g
- Silicone volatil autoémulsionnant "DOW CORNING Q2-3225C"................. 39,29g
- Huile de paraffine épaisse............. 0,78g
- Complexe anthraline-cholestérol........ 5,68g
- Eau distillée......................... 15,74g

Après avoir mélangé le silicone élastomère avec le silicone volatil, on ajoute lentement sous agitation l'eau distillée de façon à obtenir une émulsion homogène.

On disperse ensuite lentement le complexe anthraline-cholestérol dans l'émulsion obtenue.

Cette émulsion reste stable au stockage, à température ambiante, pendant une période de temps supérieure à 12 mois.

EXEMPLE 3 - FILM

- Complexe anthraline-25-hydroxy cholestérol........ 5,68g
- Silicone élastomère "Dow Corning MDX-4-4210"...................................... 85g
- Agent vulcanisant "Dow Corning MDX-4-4210 Curing Agent"......................... 9,32g

Ce film est obtenu selon le procédé suivant:

On mélange dans un mortier le silicone base et son agent vulcanisant puis l'on disperse lentement le complexe anthraline-cholestérol, tout en mélangeant à l'aide d'une spatule.

Après homogénéisation au tricylindre, on dépose environ 500mg de la préparation ainsi obtenue, sur une plaque de verre. On dégaze à la pompe à vide à membrane, puis on complète le dégazage à la pompe à palettes.

Lorsque la préparation ne présente plus de bulles d'air, on applique progressivement une deuxième plaque de verre sur la précédente et on presse les deux plaques l'une contre l'autre à l'aide d'un système de serrage approprié.

On place alors l'ensemble à l'étuve à 75°C pendant 45 minutes puis on laisse refroidir et décolle le film.

EXEMPLE 4 - FORME SECHE

Une forme sèche peut être obtenue en mélangeant les composés suivants:

- Complexe anthraline-cholestérol....... 5,68g

- Peroxyde de zinc........................ 5g
- Silice pure précipitée............qsp 100g

EXEMPLE 5 - SPRAY POUDRE

La préparation d'un spray sec peut être obtenue à partir des composés suivants:

- Complexe anthraline-25-hydroxy cholestérol....... 1,14g
- Silice colloïdale....................
- Oxyde de zinc........................ } 20g
- Talc.................................
- Gaz propulseurs.............................. qsp 150g
  trichlorofluorométhane
  dichlorofluorométhane

**Revendications**

1. Complexe à base d'anthraline et d'un stérol, caractérisé par le fait que son point de fusion est inférieur de 10 à 25°C à celui du constituant du complexe fondant à la température la plus basse et que la proportion d'anthraline varie entre 10 et 90% molaire.

2. Complexe selon la revendication 1, caractérisé par le fait que le stérol est choisi parmi:
(i) le cholestérol
(ii) les dérivés mono et polyhydroxylés ou carboxylés sur la chaine latérale en $C_{17}$ du cholestérol et leurs alkyl-esters inférieurs,
(iii) les dérivés obtenus par isomérisation et/ou création d'insaturations dans la chaîne latérale en $C_{17}$ du cholestérol, et
(iv) les esters jusqu'à 12 atomes de carbone et les éthers ayant jusqu'à 6 atomes de carbone en position $C_3$ du cholestérol.

3. Complexe selon les revendications 1 et 2, caractérisé par le fait qu'il est choisi parmi les suivants:
(i) anthraline (0,26%M) - cholestérol (0,74%M),
(ii) anthraline (0,70%M) - 25-hydroxy-cholestérol (0,30%M),
(iii) anthraline (0,30%M) - stigmastérol (0,70%M),
(iv) anthraline (0,42%M) - acétate de cholestérol (0,58%M).
(v) Anthraline (0,17%M) -20-hydroxy-cholestérol (0,83%M), et
(vi) Anthraline (0,35%M)-Ester méthylique de l'acide (3-hydroxychol-5-ène)-24-oïque (0,65%M)

4. Complexe selon la revendication 3, caractérisé par le fait qu'il est le complexe anthraline-cholestérol et a un point de fusion de 132° ± 2°C.

5. Complexe selon la revendication 3, caractérisé par le fait qu'il est le complexe anthraline - 25-hydroxy-cholestérol et a un point de fusion de 157° ± 2°C.

6. Complexe selon la revendication 3, caractérisé par le fait qu'il est le complexe anthraline-stigmastérol et a un point de fusion de 150° ± 2°C.

7. Complexe selon la revendication 3, caractérisé par le fait qu'il est le complexe anthraline-acétate de cholestérol et a un point de fusion de 102° ± 2°C.

8. Complexe selon la revendication 3, caractérisé par le fait qu'il est le complexe Anthraline-20-hydroxy-cholestérol et a un point de fusion de 118° ± 2°C.

9. Complexe selon la revendication 3, caractérisé par le fait qu'il est le complexe Anthraline - Ester méthylique de l'acide (3-hydroxychol-5-ène)-24-oïque et a un point de fusion de 126° ± 2°C.

10. Procédé de préparation des complexes selon l'une quelconque des revendications 1 à 9, caractérisé par le fait qu'il consiste à chauffer, sous agitation et sous azote, le mélange d'anthraline et du stérol à une température voisine de celle du point de fusion du composé qui a la température de fusion la plus élevée, à maintenir cette température pendant environ 10 à 20 minutes et à isoler le complexe sous forme d'une poudre.

11. Composition pharmaceutique ou cosmétique, caractérisée par le fait qu'elle contient, en tant que substance active, au moins un complexe antraline/stérol tel que revendiqué selon l'une quelconque des revendications 1 à 9, dans un véhicule pharmaceutiquement ou cosmétiquement acceptable.

12. Composition selon la revendication 11, caractérisée par le fait que le complexe anthraline/stérol est présent en une proportion comprise entre 0,5 et 10% en poids.

13. Composition selon la revendication 11 ou 12, caractérisée par la fait qu'elle se présente sous forme d'un onguent, d'une émulsion eau-dans-l'huile, d'un film ou sous forme sèche.

14. Composition selon la revendication 13, caractérisée par le fait que l'émulsion eau-dans-l'huile contient un silicone élastomère du type polyvinyldiméthylsiloxane associé à une charge de silice en tant qu'agent épaississant.

**Patentansprüche**

1. Komplex auf Basis von Anthralin und einem Sterol, **dadurch gekennzeichnet**, daß sein Schmelzpunkt unter 10 bis 25°C desjenigen liegt, bei dem der Komplexbestandteil mit der niedrigsten Schmelztemperatur schmilzt und daß der Mengenanteil Anthralin zwischen 10 und 90 Mol-% schwankt.

2. Komplex nach Anspruch 1, **dadurch gekennzeichnet,** daß das Sterol ausgewählt ist aus

(I) Cholesterol

(II) Mono- und Polyhydroxyderivate oder -Carboxylate in einer Seitenkette am $C_{17}$ des Cholesterols und ihre niederen Alkylester.

(III)Derivate, erhalten durch Isomerisierung und/oder Schaffung ungesättigter Stellen in der Seitenkette am $C_{17}$ des Cholesterols und

(IV) Ester bis 12 Kohlenstoffatome und deren Ether bis 6 Kohlenstoffsatomen in $C_3$-Stellung des Cholesterols.

3. Komplexe nach Anspruch 1 und 2, **dadurch gekennzeichnet**, daß er ausgewählt ist aus folgenden Bestandteilen:

(I) Anthralin (0,26 Mol-%)-Cholesterol (0,74 Mol-%)

(II) Anthralin (0,70 Mol-%)-25-Hydroxy-cholesterol (0,30 Mol-%)

(III) Anthralin (0,30 Mol-%)-Stigmasterol (0,70 Mol-%)

(IV) Anthralin (0,42 Mol-%)-Cholesterolacetat (0,58 Mol-%)

(V) Anthralin (0,17 Mol-%)-20-Hydroxycholesterol (0,83 Mol-%)

(VI) Anthralin 0,35 Mol-%)-Methylester der (3-Hydroxychol-5-en)-25-säure (0,65 Mol-%)

4. Komplex nach Anspruch 3, **dadurch gekennzeichnet**, daß der Anthralin-Cholesterolkomplex einen Schmelzpunkt von 132 ± 2°C hat.

5. Komplex nach Anspruch 3, **dadurch gekennzeichnet**, daß der Anthralin-24-Hydroxycholesterolkomplex einen Schmelzpunkt von 157 ± 2°C hat.

6. Komplex nach Anspruch 3, **dadurch gekennzeichnet**, daß der Anthralin-Stigmasterolkomplex einen Schmelzpunkt von 150 ± 2°C hat.

7. Komplex nach Anspruch 3, **dadurch gekennzeichnet,** daß der Anthralin-Cholesterolacetat-Komplex einen Schmelzpunkt von 102 ± 2°C hat.

8. Komplex nach Anspruch 3, **dadurch gekennzeichnet**, daß der Anthralin-20-Hydroxycholesterol-Komplex einen Schmelzpunkt von 118 ± 2°C hat.

9. Komplex nach Anspruch 3, **dadurch gekennzeichnet**, daß der Anthralin-Methylester der (3 Hydroxychol-5-en)24-säure-Komplex einen Schmelzpunkt von 126 ± 2°C hat.

10. Verfahren zur Herstellung von Komplexen nach den Ansprüchen 1 bis 9, **dadurch gekennzeichnet**, daß es darin besteht, ein Gemisch von Anthralin und dem Sterol unter Rühren und unter Stickstoffatmosphäre auf eine Temperatur zu erwärmen, die nahe dem Schmelzpunkt der Mischung liegt, die eine höhere Schmelztemperatur hat und daß man diese Temperatur 10 bis 20 Minuten aufrechterhält und den gebildeten Komplex als Pulver isoliert.

11. Pharmazeutische oder kosmetische Zubereitung, **dadurch gekennzeichnet**, daß sie als aktiven Bestandteil mindestens einen Anthralin-Sterol-Komplex nach den Ansprüchen 1 bis 9 zusammen mit einem pharmazeutischen oder kosmetischen unbedenklichen Träger enthält.

12. Zubereitungen nach Anspruch 11, **dadurch gekennzeichnet**, daß der Anthralin-Sterol-Komplex in einer Menge zwischen 0,5 und 10 Gew.-% vorhanden ist.

13. Zubereitung nach Anspruch 11 oder 12, **dadurch gekennzeichnet**, daß sie in Form einer Salbe, einer Wasser-in-Öl-Emulsion, eines Films oder in trockener Form vorliegt.

14. Zubereitung nach Anspruch 13, **dadurch gekennzeichnet**, daß die Wasser-in-Öl-Emulsion ein elastomeres Silikon des Types Polyvinyldimethylsiloxan zusammen mit einer Menge Siliciumdioxid als Verdickungsmittel enthält.

## Claims

1. Complex based on anthralin and a sterol, characterized in that its melting point is 10 to 25°C lower than that of the constituent of the complex melting at the lower temperature, and in that the proportion of anthralin varies between 10 and 90 mol %.

2. Complex according to Claim 1, characterized in that the sterol is selected from:
(i) cholesterol
(ii) derivatives mono- and polyhydroxylated or carboxylated on the side chain at C-17 of cholesterol and their lower alkyl esters,
(iii) derivatives obtained by isomerization and/or creation of unsaturations in the side chain at C-17 of cholesterol, and
(iv) esters up to 12 carbon atoms and ethers having up to 6 carbon atoms at position C-3 of cholesterol.

3. Complex according to Claims 1 and 2, characterized in that it is selected from the following:
(i) anthralin (0.26 mol %)/cholesterol (0.74 mol %),
(ii) anthralin (0.70 mol %)/25-hydroxycholesterol (0.30 mol %),
(iii) anthralin (0.30 mol %)/stigmasterol (0.70 mol %),
(iv) anthralin (0.42 mol %)/cholesterol acetate (0.58 mol %).
(v) Anthralin (0.17 mol %)/20-hydroxycholesterol (0.83 mol %), and
(vi) Anthralin (0.35 mol %)/3-Hydroxy-5-cholen-25-oic (sic) acid methyl ester(0.65 mol %).

4. Complex according to Claim 3, characterized in that it is the anthralin/cholesterol complex and has a melting point of 132° ± 2°C.

5. Complex according to Claim 3, characterized in that it is the anthralin/24-hydroxycholesterol complex and has a melting point of 157° ± 2°C.

6. Complex according to Claim 3, characterized in that it is the anthralin/stigmasterol complex and has a melting point of 150° ± 2°C.

7. Complex according to Claim 3, characterized in that it is the anthralin/cholesterol acetate complex and has a melting point of 102° ± 2°C.

8. Complex according to Claim 3, characterized in that it is the Anthralin/20-hydroxycholesterol complex and has a melting point of 118° ± 2°C.

9. Complex according to Claim 3, characterized in that it is the Anthralin/3-hydroxy-5-cholen-24-oic acid methyl ester complex and has a melting point of 126° ± 2°C.

10. Process for preparing complexes according to any one of Claims 1 to 9, characterized in that it consists in heating, with stirring and under nitrogen, the mixture of anthralin and sterol to a temperature in the region of that of the melting point of the compound which has the higher melting point, in maintaining this temperature for approximately 10 to 20 minutes and in isolating the complex in the form of a powder.

11. Pharmaceutical or cosmetic composition, characterized in that it contains, as active substance, at least one anthralin/sterol complex as claimed according to any one of Claims 1 to 9, in a pharmaceutically or cosmetically acceptable vehicle.

12. Composition according to Claim 11, characterized in that the anthralin/sterol complex is present in a proportion of between 0.5 and 10 % by weight.

13. Composition according to Claim 11 or 12, characterized in that it is presented in the form of an ointment, a water-in-oil emulsion or a film, or in dry form.

14. Composition according to Claim 13, characterized in that the water-in-oil emulsion contains a silicone elastomer of the polyvinyldimethylsiloxane type, combined with a silica filler as a thickening agent.